(19) Europäisches Patentamt
European Patent Office
Office européen des brevets

(11) Veröffentlichungsnummer: **0 337 928**
**A1**

(12) # EUROPÄISCHE PATENTANMELDUNG

(21) Anmeldenummer: **89730086.9**

(22) Anmeldetag: **31.03.89**

(51) Int. Cl.4: **C 07 D 401/06**
A 61 K 31/415, A 61 K 31/44,
A 61 K 31/38, C 07 D 409/06

(30) Priorität: **31.03.88 DE 3811574**

(43) Veröffentlichungstag der Anmeldung:
**18.10.89 Patentblatt 89/42**

(84) Benannte Vertragsstaaten:
**AT BE CH DE ES FR GB GR IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Strehlke, Peter, Dr.**
**Droysenstrasse 10A**
**D-1000 Berlin 12 (DE)**

**Bohlmann, Rolf, Dr.**
**Kühler Weg 6a**
**D-1000 Berlin 19 (DE)**

**Henderson, David, Dr.**
**Jahnstrasse 17**
**D-1000 Berlin 28 (DE)**

**Nishino, Yukishige, Dr.**
**Lanzendorfer Weg 14**
**D-1000 Berlin 22 (DE)**

(54) **N-substituierte Imidazole, Verfahren zu ihrer Herstellung sowie ihre Verwendung in Arzneimitteln.**

(57) Die Erfindung betrifft N-substituierte Imidazole der allgemeinen Formel I, Verfahren zu ihrer Herstellung sowie ihre Verwendung in Arzneimitteln.

Die erfindungsgemäßen Verbindungen besitzen aromatasehemmende Eigenschaften.

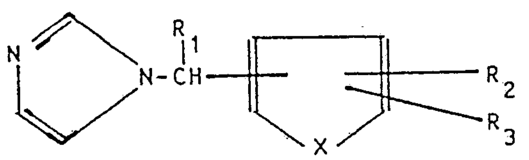

worin

$R_1$ ein Wasserstoffatom, einen gesättigten oder ungesättigten, gerad- oder verzweigtkettigen Kohlenwasserstoffrest mit 1 bis 10 Kohlenstoffatomen oder einen cyclischen Kohlenwasserstoffrest mit 3 bis 9 Kohlenstoffatomen oder einen Cycloalkylalkylrest mit 4 bis 12 Kohlenstoffatomen oder einen Arylalkylrest mit 7 bis 10 Kohlenstoffatomen,

$R_2$ ein Wasserstoffatom, ein Halogenatom, eine Cyanogruppe, eine gegebenenfalls substituierte Methylgruppe, eine gegebenenfalls derivatisierte Carboxylgruppe, eine gegebenenfalls substituierte Alkanoylgruppe mit 2 bis 10 Kohlenstoffatomen, eine gegebenenfalls substituierte Benzoylgruppe,

$R_3$ ein Wasserstoffatom oder eine gegebenenfalls substituierte Benzyloxygruppe

und

X -CH=N- ;

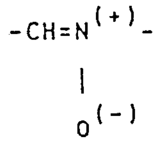

oder S ;

bedeutet,

sowie die Verbindungen in Form ihrer Salze.

EP 0 337 928 A1

## Beschreibung

### N-substituierte Imidazole, Verfahren zu ihrer Herstellung sowie ihre Verwendung in Arzneimitteln

Die Erfindung betrifft N-substituierte Imidazole, Verfahren zu ihrer Herstellung sowie ihre Verwendung in Arzneimitteln.

Die erfindungsgemäßen Verbindungen werden durch die allgemeinen Formel I

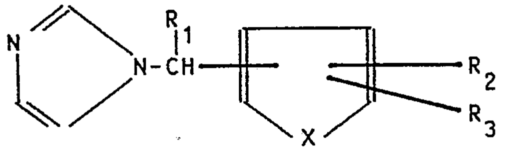

(I)

beschrieben,
worin
$R_1$ ein Wasserstoffatom, einen gesättigten oder ungesättigten, gerad- oder verzweigtkettigen Kohlenwasserstoffrest mit 1 bis 10 Kohlenstoffatomen oder einen cyclischen Kohlenwasserstoffrest mit 3 bis 9 Kohlenstoffatomen oder einen Cycloalkylalkylrest mit 4 bis 12 Kohlenstoffatomen oder einen Arylalkylrest mit 7 bis 10 Kohlenstoffatomen,
$R_2$ ein Wasserstoffatom, ein Halogenatom, eine Cyanogruppe, eine gegebenenfalls substituierte Methylgruppe, eine gegebenenfalls derivatisierte Carboxylgruppe, eine gegebenenfalls substituierte Alkanoylgruppe mit 2 bis 10 Kohlenstoffatomen, eine gegebenenfalls substituierte Benzoylgruppe,
$R_3$ ein Wasserstoffatom oder eine gegebenenfalls substituierte Benzyloxygruppe
und
X -CH=N- ;

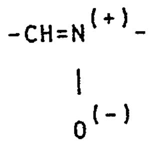 oder S

bedeutet.

Die Verbindungen der allgemeinen Formel I können auch in Form ihrer Salze vorliegen.

Die als Rest $R_1$ infrage kommenden gesättigten oder ungesättigten, gerad- oder verzweigtkettigen Kohlenwasserstoffreste mit 1 bis 10 Kohlenstoffatomenm sind beispielsweise der Methyl-, Ethyl-, Propyl-, Propenyl-, Isopropyl-, Butyl-, Hexyl-, Octyl- und Decylrest, wobei der Propylrest bevorzugt ist. Als cyclische Kohlenwasserstoffreste mit 3 bis 9 Kohlenstoffatomen sind beispielsweise der Cyclopentyl- und Cyclohexylrest zu nennen. Als Cycloalkylalkylreste mit 4 bis 12 Kohlenstoffatomen sind beispielsweise der Cyclopentylmethyl- und Cyclohexylmethylrest zu nennen. Als Arylalkylrest mit 7 bis 10 Kohlenstoffatomen ist beispielsweise der Benzylrest zu nennen.

Die als Rest $R_2$ infrage kommenden Halogenatome sind Fluor, Chlor, Brom und Jod, wobei Brom bevorzugt ist. Als Substituenten der Methylgruppe, wenn für den Rest $R_2$ eine substituierte Methylgruppe steht, sind beispielsweise Halogene, Ether-und Aminreste, insbesondere jedoch Hydroxyl-, Cyano-, Methoxy- und Pyrrolidinylreste, zu nennen.

Weiter hat $R_2$ die Bedeutung einer gegebenenfalls derivatisierten Carboxylgruppe, wobei als Derivate beispielsweise Carbonsäureester und Carbonsäureamide zu nennen sind. Als besonders geeignet haben sich Methyl-, Ethyl- und Butylester sowie Carbonsäureamid, Isopropylamid, Anilid und Pyrrolidinylamid erwiesen.

Weiter hat $R_2$ die Bedeutung einer gegebenenfalls substituierten Alkanoylgruppe mit 2 bis 10 Kohlenstoffatomen. Beispielsweise kann diese Alkanoylgruppe gerad-und verzweigtkettig sein und an beliebigen Stellen der Kette ein- oder mehrfach substituiert sein. Als Substituenten der Alkanoylgruppe sind Halogenatome sowie Methoxy-, Ethoxy-, Amino-, Hydroxyl- und Cyanogruppen geeignet. Als bevorzugte Alkanoylgruppen sind Acetyl-, Propinoyl-, Butyryl-, Valeryl- und Caproylgruppen zu nennen.

Als Substituenten der weiter als Rest $R_2$ infrage kommenden substituierten Benzoylgruppe sind Halogenatome, $C_{1-4}$-Alkyl-, Methoxy-, Ethoxy-, Amino-, Hydroxyl- und Cyanogruppen, wobei ein oder mehrere Substituenten an beliebigen Stellen der Benzoylgruppe stehen können, geeignet.

Die als Rest $R_3$ infrage kommenden gegebenenfalls substituierten Benzyloxygruppen können als Substituenten am Aromaten ein oder mehrere Halogenatome sowie Hydroxyl-, Ether-, Amino- und Cyanogruppen enthalten. Als substituierte Benzyloxygruppen haben sich die 3-Brom-, 4-Brom-, 4-Chlor-, 2,3-, 2,4-, 4,5-und 4,6-Dichlorbenzyloxygruppen als besonders geeignet gezeigt.

Als Rest X finden solche zweiwertigen Gruppen Anwendung, bei denen sich als Aromat ein Pyridin-, ein Pyridin-N-Oxyd- oder ein Thiophenring ausbilden kann.

Die Reste $R_2$ und $R_3$ können an allen infrage kommenden Positionen des aromatischen Ringes stehen.

Als mögliche Salze der Verbindungen der allgemeinen Formel I sind physiologisch verträgliche Salze von

organischen oder auch von anorganischen Säuren zu nennen. Als Salze sind besonders geeignet das Malonat, das Succinat, das Hydrochlorid und das Hydrobromid.

Die Verbindungen der allgemeinen Formel I sind Inhibitoren der Östrogenbiosynthese (Aromatasehemmer). Sie sind damit zur Behandlung von Krankheiten geeignet, die durch Östrogene bedingt oder von Östrogenen abhängig sind. So sind sie geeignet zur Behandlung von östrogeninduzierten oder -stimulierten Tumoren, wie zum Beispiel Mammakarzinom oder Prostatahyperplasie (The Lancet, 1984, S. 1237-1239).

Die erfindungsgemäßen Verbindungen sind auch wertvoll zur Beeinflussung der Fertilität. So kann eine männliche Infertilität, die aus erhöhten Östrogenspiegeln resultiert, mit den neuen Wirkstoffen behoben werden.

Ferner können die Verbindungen bei Frauen im fortpflanzungsfähigen Alter als Antifertilitätsmittel verwendet werden, um Ovulationen durch Östrogenentzug zu hemmen.

Wahrscheinlich sind Aromatasehemmer auch zur Behandlung des drohenden Herzinfarktes geeignet, da erhöhte Östrogenspiegel beim Mann einem Herzinfarkt vorangehen können (US-Patent 4.289.762).

Bekannte, aromatasehemmende Wirkung aufweisende Substanzen sind neben Steroiden auch nichtsteroidale Substanzen; wie die zum Beispiel in den europäischen Patentanmeldungen, Veröffentlichungsnummern 0165777 bis 0165784 beschriebenen diversen Stickstoffheterocyclen, die in J.Med.Chem. 1986 29, Seiten 1362-1369 beschriebenen substituierten Glutarsäureimide, die in der europäischen Patentanmeldung, Veröffentlichungsnummer 0165904, beschriebenen substituierten Imidazo-benzole und die in der europäischen Patentanmeldung, Veröffentlichungsnummer 0236940, beschriebenen substituierten heterocyclisch substituierten Toluolnitrile.

Im Vergleich zu anderen bekannten nichtsteroidalen Aromatasehemmern sind die Verbindungen der vorliegenden Anmeldung dadurch gekennzeichnet, daß sie das Enmzymsystem der Aromatase stärker selektiv hemmen, ohne andere Enzymsysteme in nennenswerterweise zu beeinträchtigen.

Die biologischen Tests A und B werden angewendet, um die Enzymaktivitäten und die Enzymselektivitäten der Verbindungen zu bestimmen.

**Test A**

**Bestimmung der Aromataseaktivität**

Die Fähigkeiten der Verbindungen, das Enzymsystem der Aromatase zu inhibieren, wird an Microsomen, erhalten aus Humanplacenta, getestet. Die Freisetzung von Tritium-markiertem Wasser ($^3H_2O$), das als Reaktionsprodukt bei der Aromatisierung von [1ß-$^3$H]-Androstendion zum Östron freigesetzt wird, wird nach der Methode von Thompson und Siiteri [J. Biol. Chem., 249, 5364-72 (1974)] gemessen. Die entsprechenden Hemmwerte ($K_i$, Aromatase) werden durch graphische Bestimmung, der Auftragung von 1/V gegen die Hemmkonzentration, gemäß der Methode von Dixon [Biochem. J. 94, 760 (1965)] bestimmt.

**Test B**

**Bestimmung der 11ß-Hydroxylaseaktivität**

Die 11ß-Hydroxylaseaktivität wird an Mitochondrien, erhalten aus Rindernebennieren, gemessen. Die Umsetzung von [1,21-$^3$H]-17$\alpha$-Hydroxy-11-desoxycorticosteron in Cortisol wird durch Dünnschichtchromatographie der entstehenden Reaktionsprodukte nach einer Methode von Mitani et al. [J. Biol. Chem., 250, 8010-15 (1975)] bestimmt. Es wird die Konzentration der untersuchten Verbindungen bestimmt, bei der die Reaktion um 50 % gehemmt wird, wobei die Substratkonzentration dem scheinbaren $K_m$,-Wert entspricht.

Am Beispiel der erfindungsgemäßen Aromatasehemmer {5-[1-(1-Imidazolyl)-butyl]-2-thienyl}-pentyl-keton (Verbindung 2) und 3-(4-Chlorbenzyloxy)-4-[1(1-imidazolyl)-pentyl-pyridin (Verbindung 3) wird gezeigt, daß diese gegenüber dem literaturbekannten Aromatasehemmer 4-[1-(1-Imidazolyl)-butyl]-benzonitril (Verbindung 1) (Europäische Patentanmeldung, Veröffentlichungsnr. 0 236 940) selektivere Wirksamkeiten (Test B), bei gleicher (Verbindung 2) und stärkerer Hemmwirkung (Verbindung 3) der Aromatase als Verbindung 1 (Test A) besitzen.

| | Test A | Test B |
|---|---|---|
| Verbindung | $K_i$, Aromatase | $IC_{50}$ 11ß-Hydroxylase |
| Verbindung 1 | 1,1 nmol/L | 94 nmol/L |
| Verbindung 2 | 1,1 nmol/L | 1 µmol/L |
| Verbindung 3 | 0,64 nmol/L | 230 µmol/L |

Die zu verabreichende Menge der Verbindungen schwanken innerhalb eines weiten Bereichs und können jede wirksame Menge abdecken. In Abhängigkeit des zu behandelnden Zustands und der Art der Verabreichung kann die Menge der verabreichten Verbindungen 0,0001-10 mg/kg Körpergewicht, vorzugsweise 0,001-1 mg/kg Körpergewicht, je Tag betragen.

Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelantine, Gleitmittel, Kieselsäure, Talkum usw. enthalten. Die einzelnen Dosierungseinheiten für die orale Applikation können beispielsweise 0,05-50 mg des Wirkstoffes (Aromataseinhibitors) enthalten.

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsmittelvermittlers, eines oberflächenaktiven Mittels, einen Suspendier- oder Emulgiergemisches verwendet. Als Beispiele für verwendete Öle sind zu nennen Olivenöl, Erdnußöl, Baumwollöl, Sojabohnenöl, Rizinusöl und Sesamöl.

Die Verbindungen lassen sich auch in Form einer Depotinjektion oder eines Implantatpräparates anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silicone, wie zum Beispiel Siliconkautschuk. Die Wirkstoffe können außerdem zur perkutanen Applikation zum Beispiel in einem Pflaster eingearbeitet werden.

Die Erfindung betrifft somit auch pharmazeutische Präparate und die Verwendung der Verbindungen zur Herstellung dieser Präparate zur Behandlung von östrogenbedingten Krankheiten.

Die Erfindung betrifft ferner Verfahren zur Herstellung von substituierten Imidazolen der allgemeinen Formel I gemäß Anspruch 1,
dadurch gekennzeichnet, daß man

a) eine Verbindung der allgemeinen Formel II

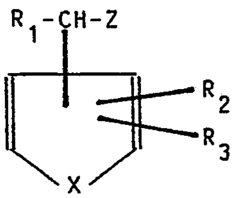

(II) ,

worin
$R_1$, $R_2$, $R_3$ und X die in Formel I genannte Bedeutung haben und
Z eine Fluchtgruppe bedeutet, mit einer Verbindung der allgemeinen Formel III

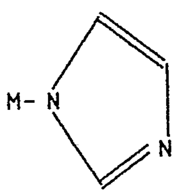

(III) ,

worin
M ein Wasserstoffatom oder ein Alkalimetallatom bedeutet,

b) eine Verbindung der allgemeinen Formel IV

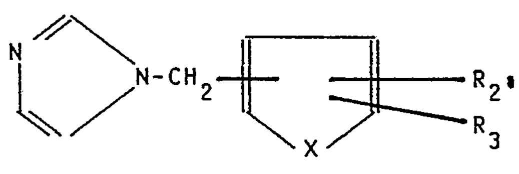

(IV) ,

worin
$R_{2'}$ dieselbe Bedeutung hat wie $R_2$ in Formel I mit der Maßgabe, daß $R_{2'}$ ein elektronenziehender, nicht deprotonierbarer Rest ist, sowie
$R_3$ und X die in Formel I genannte Bedeutung haben,
mit einer Verbindung der allgemeinen Formel V
$R_1$-Z (V),
worin
$R_1$ die in Formel I genannte Bedeutung hat
und
Z eine Fluchtgruppe bedeutet,

c) eine Verbindung der allgemeinen Formel V1

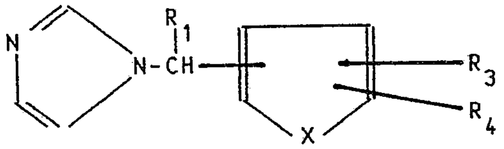

(VI) ,

worin
$R_1$, $R_3$ und X die in Formel I genannte Bedeutung haben und
$R_4$ ein Halogenatom bedeutet,
mit einem Metallcyanid in die Cyanoverbindung, gegebenenfalls die Cyanoverbindung mit einer metallorganischen Verbindung in das Alkanoyl- oder gegebenenfalls substituierte Benzoylderivat, gegebenenfalls die Cyanoverbindung hydrolytisch in die Carboxylverbindung oder in das Carbonsäureamid, gegebenenenfalls die Carboxylverbindung mit einem Alkohol zum Ester, gegebenenenfalls den Ester mit einem Reduktionsmittel zur Hydroxymethylverbindung, gegebenenfalls die Hydroxymethylverbindung mit einem Halogenierungsmittel zur Halogenmethylverbindung, gegebenenfalls die Halogenmethylverbindung mit Cyanid zur Cyanomethylverbindung, gegebenenfalls die Carboxylverbindung mit einem Halogenierungsmittel in das Carbonsäurehalogenid und gegebenenfalls das Carbonsäurehalogenid mit Ammoniak in das Carbonsäureamid oder mit Aminen in die substituierten Carbonsäureamide,

d) eine Verbindung der allgemeinen Formel VII

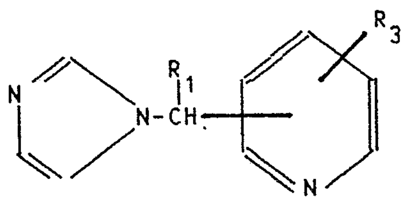

(VII) ,

worin
$R_1$ und $R_3$ die in Formel I genannte Bedeutung haben,
mit einem Oxidationsmittel zum Pyridin-N-Oxid und gegebenenfalls mit einem Elektrophil und einem Metallcyanid oder mit Trimethylsilylcyanid zur Cyanoverbindung
umsetzt.

Für die unter a) genannten Umsetzungen eignen sich als Fluchtgruppen Z literaturbekannte, leicht substituierbare Gruppen. Als solche zur Fluchtgruppenfähigkeit geeignete funktionelle Gruppen sind beispielsweise die benzylische Hydroxyl-, die Mesyl-, die Tosyl-, die Triflat- und die Acetylgruppe zu nennen. Als geeignete Gruppen haben sich auch die Halogene Chlor und Brom erwiesen. Die anzuwendenden Methoden sind u.a. in Eur.J.Med.Chem. 1979, Seiten 231-237, beschrieben.

Als Substituenten M der allgemeinen Formel III kommen Wasserstoff und Alkalimetallatome infrage. Als Alkalimetallatome sind Lithium, Natrium und Kalium bevorzugt.

Die Reaktionen die unter a) aufgeführt sind, die Umsetzungen von Verbindungen der allgemeinen Formel II mit Verbindungen der allgemeinen Formel III, lassen sich in allen inerten organischen Lösungsmitteln durchführen. Als Lösungsmittel sind beispielsweise Dimethylformamid, Dimethylsulfoxyd und diverse Ether (zum Beispiel Tetrahydrofuran, Dioxan und Diethylether) geeignet.
Die Umsetzung der Verbindungen der Formel II mit Imidazol (Formel III, M gleich Wasserstoff) kann auch ohne ein Lösungsmittel und bei Temperaturen zwischen dem Schmelzpunkt und dem Siedepunkt von Imidazol, vorzugsweise zwischen 100 °C und 200 °C, vorgenommen werden.

Die Herstellung der Verbindungen der allgemeinen Formel I kann auch gemäß Verfahren b) durchgeführt werden. Es erfolgt dabei die Umsetzung von Verbindungen der allgemeinen Formel IV mit Verbindungen der allgemeinen Formel V. Als Fluchtgruppe Z der Verbindungen der allgemeinen Formel V sind alle gängigen Fluchtgruppen geeignet, zum Beispiel die bei a) genannten.

Zur Herstellung der Verbindungen der allgemeinen Formel I nach dem Verfahren b) wird mit Basen aus den Verbindungen der allgemeinen Formel IV das entsprechende Benzylanion erzeugt und mittels Standardmethoden mit den Verbindungen der allgemeinen Formel V umgesetzt. Die benzylische $-CH_2$-Gruppe in den Verbindungen der allgemeinen Formel IV läßt sich mittels Basen, zum Beispiel durch Umsetzung mit tertiären Aminen, Natriumhydrid, Lithiumhydrid, Lithiumdiisopropylamid und Magnesiumhydrid deprotonieren und mit Verbindungen der allgemeinen Formel V zur Reaktion bringen. Das Verfahren b) findet vorzugsweise Anwendung, wenn $R_2$ eine Akzeptorgruppe, beispielsweise eine Cyano- oder eine Benzoylgruppe, darstellt.

Die erfindungsgemäßen Verbindungen der allgemeinen Formel I lassen sich auch nach dem Verfahren c) herstellen. Dabei werden Verbindungen der allgemeinen Formel VI nach Standardmethoden umgesetzt. Als mögliche Reste $R_4$ eignen sich Halogenatome, zum Beispiel Chlor und Brom.

Durch nucleophile Substitution eines Metallcyanids, beispielsweise Kupfer-(I)-cyanid, lassen sich die Halogen- in die entsprechenden Cyanoverbindungen überführen.

Die erhaltenen Cyanoverbindungen lassen sich weiter mit metallorganischen Verbindungen, beispielsweise Grignardverbindungen, in die Alkanoyl- oder Benzoylverbindungen überführen. Werden die Cyanoverbindungen mit speziellen Reduktionsmitteln, wie Natrium-triethoxyaluminiumhydrid, Lithium-triethoxyaluminiumhydrid, Natriumaliminiumhydrid oder Aluminium-Nickel-Legierungen in Säuren, vorzugsweise in Ameisensäure, reduziert, so werden die entsprechenden Aldehyde erhalten.
Die Cyanoverbindungen lassen sich unter hydrolytischen Bedingungen in die entsprechenden Carbonsäuren oder Carbonsäureamide überführen.
Die erhaltenen Carboxylverbindungen lassen sich beliebig mit Alkoholen unter Standardbedingungen der Veresterung in die entsprechenden Carbonsäureester umsetzen.

Die Carbonsäureester können wiederum Ausgangsprodukte für eine Reihe von Folgeprodukten sein. So lassen sich die Ester mit Reduktionsmitteln, beispielsweise Lithiumaluminiumhydrid, in die Hydroxymethylderivate überführen.

Die Hydroxymethylderivate können mit Halogenierungsmitteln, beispielsweise Thionylchlorid, in die Halogenmethylderivate, beispielsweise in die Chlormethylderivate, umgesetzt werden. Die Halogenmethylverbindungen können weiter durch nucleophile Substitution, beispielsweise durch Umsetzung mit Alkalicyanid, Ammoniak oder Aminen, in die Cyanomethyl-, Aminomethyl- oder Alkylaminomethylverbindungen überführt werden.

Die erhaltenen Carboxylverbindungen lassen sich mit Halogenierungsmitteln, beispielsweise Thionylchlorid, in die Säurehalogenide, beispielsweise Säurechloride, überführen. Die Carbonsäurehalogenide sind wiederum Ausgangsmaterial für eine Reihe von Folgeprodukten. Durch Umsetzung mit Ammoniak oder Aminen sind die entsprechenden Amide erhältlich.

Verbindungen der allgemeinen Formel I, bei denen X -CH=N- oder -CH=NO-bedeutet, lassen sich auch nach dem Verfahren d) herstellen. Dabei werden Verbindungen der allgemeinen Formel VII mit einem Oxidationsmittel, beispielsweise Wasserstoffperoxid, in die entsprechenden Pyridin-N-oxide überführt. Werden diese Pyridin-N-oxide nach den in Heterocycles 22 (1984) S. 2375 beschriebenen Methoden umgesetzt, so werden Verbindungen der allgemeinen Formel I erhalten, bei denen $R_2$ für eine Cyanogruppe steht. Die Cyanoverbindungen lassen sich, wie unter Verfahren c) beschrieben, in Folgeprodukte überführen.

## Beispiele

### Beispiel 1

2-[1-(1-Imidazolyl)-butyl]-5-brom-thiophen

Ausgehend vom 5-Brom-thiophen-2-carbaldehyd wird durch Umsetzung mit Propylmagnesiumbromid ein sekundärer Alkohol erhalten, der sich weiter durch Umsetzung mit Thionylchlorid in das 1-(5-Brom-2-thienyl)-1-chlor-propan überführen läßt. 0,122 g Imidazol werden in 3 ml Dimethylformamid gelöst und mit 0,054 g Natriumhydrid (80 %ig) versetzt. Es wird 1 Stunde bei Raumtemperatur gerührt. Zu dieser Lösung werden 0,360 g 1-(5-Brom-2-thienyl)-1-chlor-propan in 2 ml Dimethylformamid zugetropft und über Nacht bei Raumtemperatur gerührt. Danach wird das Reaktionsgemisch in 100 ml 2 n Salzsäure gegossen und mit Ether extrahiert. Die Wasserphase wird mit Kaliumcarbonat alkalisch gestellt und anschließend mit Essigester extrahiert. Die Essigesterphase wird im Vakuum eingeengt und der Rückstand am Kugelrohr destilliert,
Siedepunkt: 180 - 200 °C/0,03 mbar. Es werden 0,70 g 2-[1-(1-Imidazolyl)-butyl]-5-brom-thiophen erhalten.

### Beispiel 2

5-[1-(1-Imidazolyl)-butyl]-thiophen-2-carbonitril

0,04 g Bromverbindung (aus Beispiel 1) werden in 2 ml N-Methylpyrrolidon gelöst, es wird 0,05 g Kupfer-I-cyanid zugegeben und unter Argon 5 Stunden bei 180 °C gerührt. Zur Reaktionslösung wird Ammoniakwasser zugegeben und anschliessend wird mit Essigester extrahiert. Die organische Phase wird im Vakuum eingeengt und das verbleibende Öl am Kugelrohr destilliert, Siedepunkt 180 °C/0,03 mbar. Es werden 0,01 g 5-[1-(1-Imidazolyl)-butyl]-thiophen-2-carbonitril erhalten.

### Beispiel 3

2-[1-(1-Imidazolyl)-butyl]-4-brom-thiophen

Aus dem käuflichen 4-Brom-thiophen-2-carbaldehyd wird durch Umsetzung mit Propylmagnesiumbromid und anschließende Chlorierung mit Thio nylchlorid das 1-(4-Brom-2-thienyl)-1-chlor-butan erhalten. Die Umsetzung der Chlorverbindung mit Imidazol erfolgt analog Beispiel 1. Es wird 2-[1-(1-Imidazolyl)-butyl]-4-brom-thiophen erhalten.

### Beispiel 4

2-[1-(1-Imidazolyl)-butyl]-thiophen-4-carbonitril

0,3 g 2-[1-(1-Imidazolyl)-butyl]-4-brom-thiophen werden in 5 ml N-Methylpyrrolidon gelöst und mit 0,3 g Kupfer-I-cyanid versetzt und 5 Stunden bei 180 °C gerührt. Anschließend wird die Reaktionsmischung in 100

ml verdünnte Ammoniaklösung gegossen und mit Essigester extrahiert. Die Essigesterphase wird im Vakuum eingeengt und am Kugelrohr destilliert, Siedepunkt: 180 °C/0,03 mbar. Es werden 0,190 g 2-[1-(1-Imidazolyl)-butyl]-thiophen-4-carbonitril erhalten.

Beispiel 5

5-[1-(1-Imidazolyl)-butyl]-2-thienyl-pentyl-keton

Zu einer aus 0,182 g Magnesium und 0,93 ml Pentylbromid in 2 ml Tetrahydrofuran bereiteten Grignard-Lösung wird eine Lösung von 0,69 g der Cyanoverbindung aus Beispiel 2 in 3 ml Tetrahydrofuran und 0,015 g Kupfer-I-bromid gegeben. Nach 20 Stunden Rühren bei Raumtemperatur wird auf 2 M Salzsäure gegeben und mit Ether extrahiert. Die Wasserphase wird mit Kaliumcarbonat alkalisiert, mit Essigester extrahiert, getrocknet, eingedampft und der Rückstand am Kugelrohr destilliert. Man erhält 0,3 g der Titelverbindung als gelbes Öl, Siedepunkt: 200 -230 °C/0,03 mbar.

Beispiel 6

5-[1-(1-Imidazolyl)-butyl]-2-thiophencarbonsäure, Hydrochlorid

1 g der Cyanoverbindung aus Beispiel 2 wird mit 5 ml konzentrierter Salzsäure 3 Stunden am Rückfluß gekocht. Nach Eindampfen im Vakuum wird mit Toluol dreimal nachdestilliert und der Rückstand mit Isopropanol ausgekocht. Es wird die Titelverbindung als zähes gelbes Harz erhalten.

Beispiel 7

5-[1-(1-Imidazolyl)-butyl]-2-thiophen-carbonsäurebutylester

1,2 g der Säure aus Beispiel 6 werden in 20 ml Butanol gelöst und die Lösung unter Eiskühlung mit Chlorwasserstoffgas gesättigt. Nach 20 Stunden wird eingedampft, zwischen Kaliumcarbonatlösung und Ether verteilt und die Etherphase abgetrennt. Nach Trocknen und Eindampfen des Lösungsmittels wird am Kugelrohr destilliert. Man erhält 200 mg der Titelverbindung,
Siedepunkt: 230 °C/0,03 mbar.

Beispiel 8

5-[1-(1-Imidazolyl)-butyl]-thiophen-2-methanol

0,2 g des Butylesters aus Beispiel 7 werden in 3 ml Tetrahydrofuran mit 0,2 g Lithiumaluminiumhydrid reduziert und in üblicher Weise aufgearbeitet. Man erhält 0,2 g der Titelverbindung als Öl.

Beispiel 9

5-[1-(1-Imidazolyl)-butyl]-thiophen-2-acetonitril

0,2 g des Alkohols aus Beispiel 8 werden mit Thionylchlorid (2 Stunden bei Raumtemperatur, Eindampfen, mit Toluol dreimal Nachdestillieren) in das Hydrochlorid der Chlormethylverbindung überführt, die in 2 ml Dimethylsulfoxid gelöst und mit 0,2 g Natriumcyanid versetzt wird. Nach 20 Stunden Rühren wird auf verdünnte Ammoniaklösung gegeben, mit Essigester extrahiert und der Essigesterextrakt getrocknet und eingedampft. Nach Destillation am Kugelrohr erhält man die Titelverbindung als Öl,
Siedepunkt: 210 - 230 °C/0,03 mbar.

Beispiel 10

5-[1-(1-Imidazolyl)-butyl]-2-thiophencarbonsäureanilid

0,7 g der Säure aus Beispiel 6 werden in üblicher Weise mit Thionylchlorid in das Säurechlorid überführt und mit 0,91 ml Anilin in 10 ml Methylenchlorid in das Amid überführt. Nach Umkristallisation aus Toluol erhält man 0,18 g der Titelverbindung vom Schmelzpunkt 165 - 166 °C.

Beispiel 11

1-(Cyclohexyl-2-thienyl-methyl)-imidazol

Aus 12,6 g Thiophen und 94 ml einer 1,6 molaren Lösung von Butyllithium in Hexan wird in 100 ml Ether in üblicher Weise 2-Thienyllithium hergestellt und mit 16,8 g Cyclohexanaldehyd bei -40 °C umgesetzt. Nach 15 Minuten bei -40 °C und einer Stunde bei Raumtemperatur wird in üblicher Weise aufgearbeitet und destilliert; man erhält 14 g Cyclohexyl-(2-thienyl)-methanol),
Siedepunkt: 166 -172 °C/20 mbar.
0,5 g davon werden in 10 ml Methylenchlorid mit 1 ml Thionylchlorid unter Eiskühlung gelöst; nach 20 Minuten bei Raumtemperatur wird im Vakuum eingedampft und mit Toluol nachdestilliert. Das Chlorid wird mit 0,55 g Imidazol 20 Stunden bei 160 °C erwärmt. Dann wird zwischen überschüssiger 2 M Salzsäure und Ether verteilt, die wässrige Phase mit Kaliumcarbonat alkalisiert und mit Essigester extrahiert. Nach Trocknen und

Eindampfen wird am Kugelrohr destilliert. Man erhält die Titelverbindung als Öl mit den Siedepunkt 230 °C / 0,03 mbar. Beim Verreiben mit Ether kristallisiert die Verbindung; Schmelzpunkt 90-92 °C.

Beispiel 12

{5-[-(1-Imidazolyl)-butyl)-2-thienyl]}-ethyl-keton
Analog Beispiel 5 wird die Cyanoverbindung mit Ethylmagnesiumbromid umgesetzt. Die Titelverbindung siedet am Kugelrohr bei 220 °C/0,03 mbar.

Beispiel 13

4-[1-(1-Imidazolyl)-butyl]-pyridin
Das für diese Reaktion notwendige Ausgangsmaterial, das 4-(1-Chlorbutyl)-pyridin, wird über die folgende Reaktionssequenz erhalten. Käufliches 4-Brom-pyridin wird durch Halogenmetallaustausch in die entsprechende 4-Lithium-pyridin-Verbindung überführt. Diese Lithium-Verbindung wird mit Butyraldehyd in den entsprechenden sekundären Alkohol überführt. Der Alkohol wird anschließend durch Umsetzung mit Thionylchlorid ins 4-(1-Chlorbutyl)-pyridin überführt.

0,68 g Imidazol werden in 5 ml Dimethylformamid gelöst, es wird 0,3 g Natriumhydrid (80 %ig) zugesetzt und 10 Minuten bei Raumtemperatur gerührt. Zu dieser Lösung wird eine Lösung von 3,87 g 4-(1-Chlorbutyl)-pyridin in 4 ml Dimethylformamid zugesetzt und 24 Stunden bei Raumtemperatur gerührt. Anschließend wird in 100 ml 10 %ige Salzsäure gegossen und mit Essigester extrahiert. Die wässrige Phase wird mit Natronlauge alkalisch gestellt (pH 13) und mit Methylenchlorid extrahiert. Die Methylenchloridlösung wird im Vakuum eingeengt und der Rückstand am Kugelrohr destilliert,
Siedepunkt: 170 -190 °C/0,04 mbar. Es werden 0,3 g 4-[1-(1-Imidazolyl)-butyl]-pyridin erhalten.

Beispiel 14

3-[1-(1-Imidazolyl)-butyl)-pyridin
Die Herstellung dieses Derivates erfolgt analog Beispiel 13. Ausgehend von 3-Brom-pyridin wird die entsprechende 3-Lithiumpyridin-Verbindung hergestellt und mit Butyraldehyd umgesetzt. Der sekundäre Alkohol wird durch Umsetzung mit Thionylchlorid in die Chlorverbindung überführt. Durch Substitution des Chlors durch Imidazol wird das 3-[1-(1-Imidazolyl)-butyl)-pyridin erhalten. Die Verbindung siedet zwischen 180 - 190 °C/0,03 mbar.

Beispiel 15

4-[1-(1-Imidazolyl)-butyl]-pyridin-N-oxid
Es werden 1,0 g 4-[1-(1-Imidazolyl)-butyl]-pyridin (Beispiel 13) in 10 ml Methylenchlorid vorgelegt. Dazu wird eine Lösung bestehend aus 2,1 g m-Chlorperbenzoesäure, gelöst in 10 ml Methylenchlorid, zugestropft. Es wird 20 Stunden bei Raumtemperatur gerührt. Anschließend werden 5 ml 3 N Salzsäure zugegeben, es wird 3 x mit Ether extrahiert, die wässrige Phase im Vakuum eingeengt. Erhalten werden 1,4 g eines hellgelben Harzes. Dieses Harz wird in 10 ml Methanol gelöst und über einen basischen Ionenaustauscher (Ionenaustauscher III der Fa. Merck) gegeben. Die methanolische Lösung wird anschließend eingeengt. Erhalten werden 0,8 g 4-[1-(1-Imidazolyl)-butyl]-pyridin-N-oxid.

Beispiel 16

3-[1-(1-Imidazolyl)-butyl]-pyridin-N-oxid
Analog der unter Beispiel 15 angegebenen Umsetzung wird das 3-[1-(1-Imidazolyl)-butyl]-pyridin mit m-Chlorperbenzoesäure zum 3-[1-(1-Imidazolyl)-butyl]-pyridin-N-oxid umgesetzt.

Beispiel 17

4-[1-(-Imidazolyl)-butyl]-2-cyan-pyridin
0,109 g 4-[1-(1-Imidazolyl)-butyl]-pyridin-N-oxid werden in 2 ml Acetonitril gelöst, dazu wird 0,150 g Trimethylsilylcyanid und 0,101 g Triethylamin gegeben. Die Reaktionslösung wird 22 Stunden bei 110 °C belassen. Die Mischung wird im Vakuum eingeengt und anschließend am Kugelrohr destilliert,
Siedepunkt: 210 - 220 °C/0,035 mbar. Es werden 0,102 g an 4-[1-(-Imidazolyl)-butyl]-2-cyano-pyridin als bräunliches Öl erhalten.

Beispiel 18

Gemisch bestehend aus 3-[1-(1-Imidazolyl)-butyl]-6-cyan-pyridin und 3-[1-(1-Imidazolyl)-butyl]-2-cyan-pyridin
0,6 g 3-[1-(1-Imidazolyl)-butyl]-pyridin-N-oxid (Beispiel 16) werden in 6 ml Acetonitril gelöst, dazu werden 0,63 ml Triethylamin und 1,0 ml Trimethylsilylcyanid gegeben. Es wird 24 Stunden bei 100 °C gerührt. Die

Aufarbeitung erfolgt analog Beispiel 17. Nach einer Kugelrohrdestillation werden 0,6 g eines braunen Öls erhalten. Siedepunkt: 210 - 240 °C/0,03 mbar. Durch NMR-Analyse wird festgestellt, daß ein Gemisch von 3-[1-(1-Imidazolyl)butyl]-6-cyanpyridin und 3-[1-(1-Imidazolyl)-butyl]-2-cyanpyridin vorliegt.

Beispiel 19

3-(4-Chlorbenzyloxy)-4-[1-(1-Imidazolyl)-pentyl]-pyridin

Ausgehend von 3-Chlor-4-pyridincarbonitril wird durch Reaktion mit 4-Chlorbenzylalkohol in Gegenwart von Natriumhydrid in Dimethylformamid das 3- (4-Chlor benzyloxy)-4-pyridincarbonitril erhalten, welches mit Butyllithium in Diethylether zu 3-(4-Chlorbenzyloxy)-4-valeryl-pyridin umgesetzt wird. Die Ketogruppe dieses 4-Valerylderivats wird mit Natriumborhydrid zur Hydroxylgruppe reduziert; erhalten wird 1-[3-(4-Chlorbenzyloxy)-4-pyridyl]-1-pentanol. Die Hydroxylgruppe des Pentanolderivats wird mit Thionylchlorid in Methylenchlorid gegen eine Chlorgruppe ausgetauscht, erhalten wird 3-(4-Chlorbenzyloxy)-4-(1-chlorpentyl)-pyridin. Durch anschließende Reaktion mit Imidazol in Gegenwart von Natriumhydrid (analog Beispiel 13) wird das 3-(4-Chlorbenzyloxy)-4-[1-(1-imidazolyl)-pentyl]-pyridin erhalten.

Beispiel 20

3- (4-Chlorbenzyloxy)-4-[1-(1-imidazolyl)-pentyl]-pyridin-N-oxid

0,624 g 3-(4-Chlorbenzyloxy)-4-[1-(1-imidazolyl)-pentyl]-pyridin werden analog Beispiel 15 mit 0,410 g m-Chlorperbenzoesäure zur Titelverbindung umgesetzt.

Beispiel 21

Gemisch bestehend aus 5-(4-Chlorbenzyloxy)-4-[1-(1-imidazolyl)-pentyl]-pyridin-2-carbonitril und 3-(4-Chlorbenzyloxy)-4-[1-(1-imidazolyl)-pentyl]-pyridin-2-carbonitril

0,578 g 3-(4-Chlorbenzyloxy)-4-[1-(1-imidazolyl)-pentyl]-pyridin-N-oxid werden mit 0,450 g Trimethylsilylcyanid gemäß Beispiel 18 umgesetzt. Es wird 0,483 g eines Gemisches bestehend aus den Titelverbindungen erhalten. Durch Hochdruckflüssigkeitschromatographie, System Methylenchlorid/Methanol (9:1), können die Verbindungen getrennt werden.

5-(4-Chlorbenzyloxy)-4-[1-(1-imidazolyl)-pentyl]-pyridin-2-carbonitril schmilzt bei 132-134 °C, 3-(4-Chlorbenzyloxy)-4-[1-(1-imidazolyl)-pentyl]-pyridin-2-carbonitril ist ein bräunliches Öl.

**Patentansprüche**

1 .) Substituierte Imidazole der allgemeinen Formel I

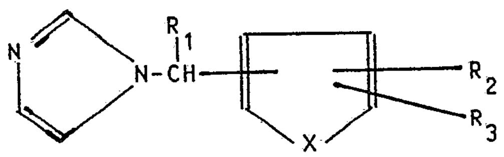

(I)

worin

$R_1$ ein Wasserstoffatom, einen gesättigten oder ungesättigten, gerad- oder verzweigtkettigen Kohlenwasserstoffrest mit 1 bis 10 Kohlenstoffatomen oder einen cyclischen Kohlenwasserstoffrest mit 3 bis 9 Kohlenstoffatomen oder einen Cycloalkylalkylrest mit 4 bis 12 Kohlenstoffatomen oder einen Arylalkylrest mit 7 bis 10 Kohlenstoffatomen,

$R_2$ ein Wasserstoffatom, ein Halogenatom, eine Cyanogruppe, eine gegebenenfalls substituierte Methylgruppe, eine gegebenenfalls derivatisierte Carboxylgruppe, eine gegebenenfalls substituierte Alkanoylgruppe mit 2 bis 10 Kohlenstoffatomen, eine gegebenenfalls substituierte Benzoylgruppe,

$R_3$ ein Wasserstoffatom oder eine gegebenenfalls substituierte Benzyloxygruppe

und

X -CH=N- ;

$-CH=N^{(+)}-$ oder S
$|$
$O^{(-)}$

bedeutet,

sowie die Verbindungen in Form ihrer Salze.

2.) Verbindungen gemäß Anspruch 1

5-[1-(1-Imidazolyl)-butyl]-thiophen-2-carbonitril
2-[1-(1-Imidazolyl)-butyl]-thiophen-4-carbonitril
2-[1-(1-Imidazolyl)-butyl]-4-brom-thiophen
2-[1-(1-Imidazolyl)-butyl]-5-brom-thiophen
{5-[1-(1-Imidazolyl)-butyl]-2-thienyl}-pentyl-keton
{5-[1-(1-Imidazolyl)-butyl]-2-thienyl}-ethyl-keton
5-[1-(1-Imidazolyl)-butyl]-2-thiophen-carbonsäure, Hydrochlorid
5-[1-(1-Imidazolyl)-butyl]-2-thiophen-carbonsäurebutylester
5-[1-(1-Imidazolyl)-butyl]-2-thiophen-carbonsäureethylester
5-[1-(1-Imidazolyl)-butyl]-thiophen-2-methanol
5-[1-(1-Imidazolyl)-butyl]-thiophen-2-acetonitril 5-[1-(1-Imidazolyl)-butyl]-thiophen-2-carbonsäureanilid
1-(Cyclohexyl-2-thienyl-methyl)-imidazol
4-[1-(1-Imidazolyl)-butyl]-pyridin
4-[1-(1-Imidazolyl)-butyl]-pyridin-N-oxid
4-[1-(1-Imidazolyl)-butyl]-2-cyan-pyridin
3-[1-(1-Imidazolyl)-butyl]-pyridin
3-[1-(1-Imidazolyl)-butyl]-pyridin-N-oxid
3-[1-(1-Imidazolyl)-butyl]-6-cyan-pyridin
3-[1-(1-Imidazolyl)-butyl]-2-cyan-pyridin
5-(4-Chlorbenzyloxy)-4-[1-(1-imidazolyl)-pentyl]-pyridin-2-carbonitril
3-(4-Chlorbenzyloxy)-4-[1-(1-imidazolyl)-pentyl]-pyridin-2-carbonitril
3-(4-Chlorbenzyloxy)-4-[1-(1-imidazolyl)-pentyl]-pyridin-N-oxid
3-(4-Chlorbenzyloxy)-4-[1-(1-imidazolyl)-pentyl]-pyridin

3.) Pharmazeutische Präparate, gekennzeichnet durch den Gehalt an mindestens einer Verbindung gemäß Anspruch 1 oder 2.

4.) Verwendung der Verbindungen nach Anspruch 1 oder 2 zur Herstellung von Präparaten zur Behandlung von östrogen-bedingten Krankheiten.

5.) Verfahren zur Herstellung von substituierten Imidazolen der allgemeinen Formel 1 gemäß Anspruch 1, dadurch gekennzeichnet, daß man

a) eine Verbindung der allgemeinen Formel II

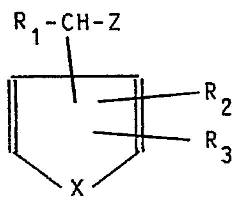

(II) ,

worin
$R_1$, $R_2$, $R_3$ und X die in Formel I genannte Bedeutung haben und
Z eine Fluchtgruppe bedeutet,
mit einer Verbindung der allgemeinen Formel III

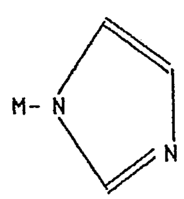

(III) ,

worin
M ein Wasserstoffatom oder ein Alkalimetallatom bedeutet,
b) eine Verbindung der allgemeinen Formel IV

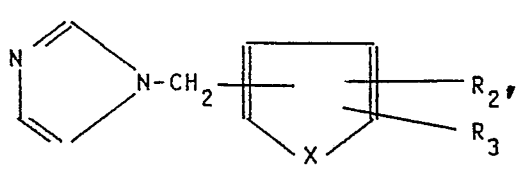

(IV) ,

worin
$R_{2'}$ dieselbe Bedeutung hat wie $R_2$ in Formel I mit der Maßgabe daß $R_{2'}$ ein elektronenanziehender, nicht deprotonierbarer Rest ist, sowie

$R_3$ und X die in Formel I genannte Bedeutung haben,
mit einer Verbindung der allgemeinen Formel V
$R_1$-Z (V),
worin
$R_1$ die in Anspruch 1 genannte Bedeutung hat
und
Z eine Fluchtgruppe bedeutet,
c) eine Verbindung der allgemeinen Formel VI

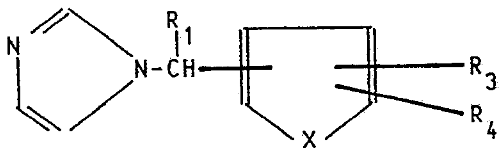

(VI)

worin
$R_1$, $R_3$ und X die in Anspruch 1 genannte Bedeutung haben und
$R_4$ ein Halogenatom
bedeutet,
mit einem Metallcyanid in die Cyanoverbindung, gegebenenfalls die Cyanoverbindung mit einer metallorganischen Verbindung in das Alkanoyl- oder gegebenenfalls substituiertes Benzoylderivat, gegebenenfalls die Cyanoverbindung hydrolytisch in die Carboxylverbindung oder in das Carbonsäureamid, gegebenenenfalls die Carboxylverbindung mit einem Alkohol zum Ester, gegebenenenfalls den Ester mit einem Reduktionsmittel zur Hydroxymethylverbindung, gegebenenfalls die Hydroxymethylverbindung mit einem Halogenierungsmittel zur Halogenmethylverbindung, gegebenenfalls die Halogenmethylverbindung mit Cyanid zur Cyanomethylverbindung, gegebenenfalls die Carboxylverbindung mit einem Halogenierungsmittel in das Carbonsäurehalogenid und gegebenenfalls das Carbonsäurehalogenid mit Ammoniak in das Carbonsäureamid oder mit Aminen in die substituierten Carbonsäureamide,
d) eine Verbindung der allgemeinen Formel VII

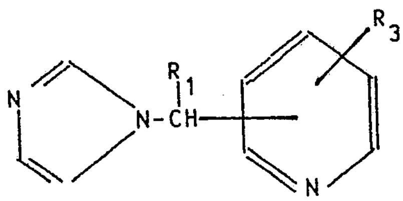

(VII),

worin
$R_1$ und $R_3$ die in Anspruch 1 genannte Bedeutung haben,
mit einem Oxidationsmittel zum Pyridin-N-oxid und gegebenenfalls mit Trimethylsilylcyanid zur Cyanoverbindung
umsetzt.

Europäisches Patentamt

# EUROPÄISCHER RECHERCHENBERICHT

Nummer der Anmeldung

EP 89 73 0086

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-3 228 266 (A. NATTERMANN & CIE. GmbH)<br>* Seite 4 * | 1-3 | C 07 D 401/06<br>A 61 K 31/415<br>A 61 K 31/44<br>A 61 K 31/38<br>C 07 D 409/06 |
| X | GB-A-2 028 317 (PFIZER LTD)<br>* Insgesamt * | 1,3,5 | |
| X | EP-A-0 003 560 (THE WELLCOME FOUNDATION LTD)<br>* Seiten 37-38,47-48,50,52 * | 1,3,5 | |
| X | EP-A-0 029 742 (PFIZER LTD)<br>* Insgesamt * | 1,3 | |
| X | EP-A-0 159 011 (DAIICHI SEIYAKU CO., LTD)<br>* Seite 29 * | 1,5 | |
| A | DE-A-3 130 250 (A. NATTERMANN & CIE GmbH) | | **RECHERCHIERTE SACHGEBIETE (Int. Cl.4)** |
| A | DE-A-3 128 277 (A. NATTERMANN & CIE GmbH) | | C 07 D 401/00<br>A 61 K 31/00<br>C 07 D 409/00 |

Der vorliegende Recherchenbericht wurde für alle Patentansprüche erstellt

| Recherchenort | Abschlußdatum der Recherche | Prüfer |
|---|---|---|
| DEN HAAG | 05-07-1989 | DE BUYSER I.A.F. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument